# EUROPEAN PATENT SPECIFICATION

(11) **EP 1 482 891 B1**
(45) Date of publication and mention of the grant of the patent: **25.02.2015**
(21) Application number: 03744421.3
(22) Date of filing: 10.03.2003
(51) Int. Cl.: A61G 7/057

(54) **INFLATABLE SUPPORT**
AUFBLASBARE STÜTZVORRICHTUNG
SUPPORT GONFLABLE

(30) Priority: 14.03.2002 GB 0205924
(43) Date of publication of application: 08.12.2004
(73) Proprietor: Huntleigh Technology Limited, Bedfordshire LU5 5XF (GB)
(72) Inventor: ROFF, Simon Michael, Dunstable, Bedfordshire LU5 4PU (GB); ROWE, Christopher, Aylesbury, Buckinghamshire HP20 2TE (GB)
(74) Representative: Thaker, Shalini
(86) International application number: PCT/GB2003/001018
(87) International publication number: WO 2003/077818

(56) References cited:
- EP-A- 0 168 213
- EP-A- 0 560 563
- EP-A- 0 853 918
- WO-A-00/32149
- WO-A-97/18737
- GB-A- 2 373 189
- US-A- 5 539 942
- US-B1- 6 212 718

## Description

This invention relates to an inflatable support, in particular an inflatable support that measures the movements of a patient supported on the support and adjusts the support provided depending on the movements detected.

There have been several techniques developed to measure body movement, patient entry and patient exit from a support using fluctuations in air pressure in the inflatable supports or inflatable bodies inserted between the patient and a supporting surface. The technique of measuring body movements by recording the air pressure in an air filled pad placed under a mattress was described by Kusunoki (1985). Another system has been developed to measure the respiratory movements of subjects by measuring pressure changes in a supporting air mattress (Hernandez 1995). US 6,036,660 describes a system that uses transducers to detect and display movement in an air-filled cell or cells between a patient and a support.

Overall, these systems give a numerical or visual display of detected movement using the fluctuation of static air in an enclosed cell or cells. Since, there is a link between the rate of spontaneous body movements and the risk of developing pressure sores (Exton-Smith et al, 1961), the information provided or displayed helps in the assessment of the risk of pressure sore development. The information can be used to increase the manual turning of the patient or to aid the decision to move the patient to another support surface. Equally, the information can be completely ignored in a busy ward. Documents WO 9718737 A1, US 5539942 A1 and US 621 2718 B1 disclose pressure pads.

The aim of this invention is to provide an inflatable support whose inflation and/or deflation regime is automatically controlled in dependence upon the movement of a patient on the support therefore patient comfort and pressure relief is automatically optimised without requiring external input from a carer or nurse. In an alternating pressure mattress, it is known that there is a compromise between an effective alternating pressure cycle used to reduce the risk of pressure sore development and the comfort experienced by the patient.

Accordingly, the invention provides an inflatable support as claimed in claim 1. Where a patient is able to move by themselves on a regular basis on an alternating mattress, the pressures at which the mattress is inflated can be adjusted to improve comfort without increasing the risk of pressure sore development, and the inflation/deflation cycle parameters can be altered to improve comfort without increasing the risk of pressure sore development. For example, the present invention can lengthen the cycle time to provide extra comfort for those patients who are making significant autonomous movements, or shorten the time for those patients that require more active pressure relief.

Preferably, a display of the patient movement is also provided. Many of the risk assessment tools (Waterlow, Norton & Braden) use movement as part of their scoring system and an accurate movement display would help the nurses select the correct support surface.

An embodiment of the present invention is described below, by way of example only, with reference to the accompanying drawings, in which:
Figure 1 is a schematic diagram of a support according to the invention;

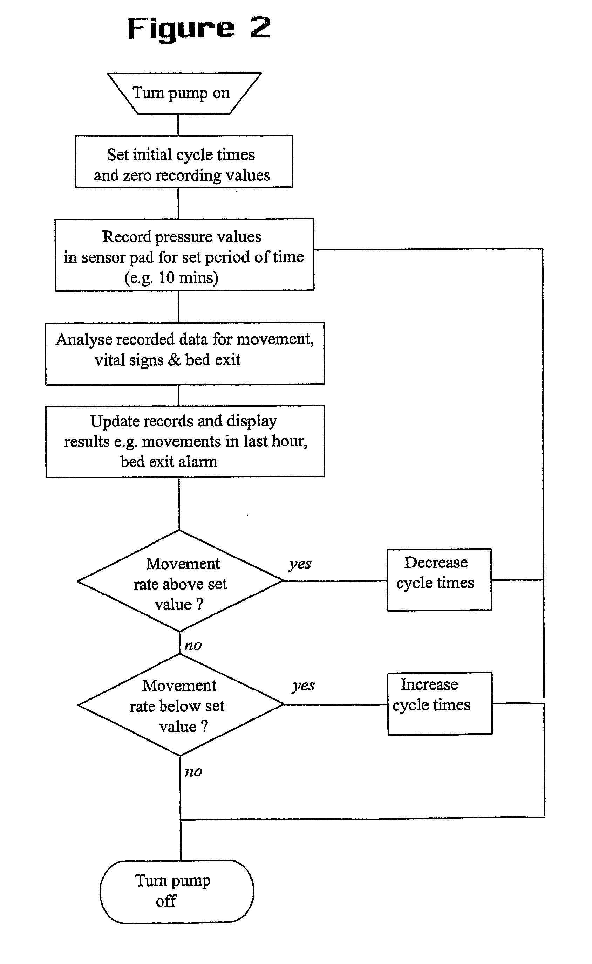

Figure 2 is a flow chart showing the control algorithm; and
Figures 3a and 3b show displays of the various body movements measured according to the invention.

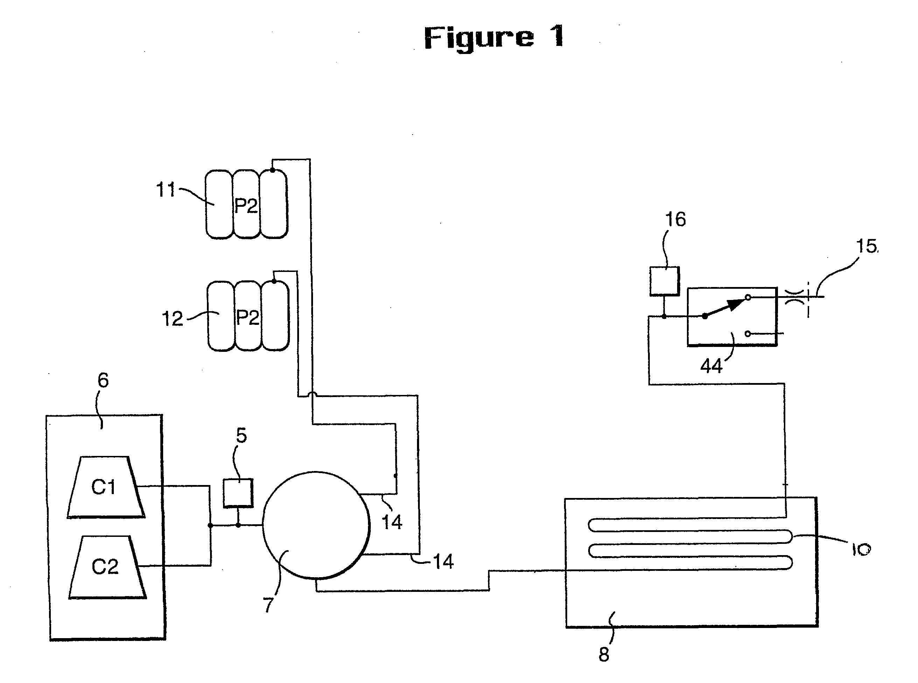

Referring to Figure 1, an alternating pad 1 is shown comprising a first set 11 and a second set 12 of alternately inflatable cells. Both sets of inflatable cells are supplied with air from a pump 6 via a rotary valve 7. A pair of air supply lines 14 lead from the rotary valve 7 to the pad 1.

A tube 10 of a sensor 8 is connected at one end to the output of the pump 6 and at the other end to a solenoid 44, pressure transducer 16 and a restrictor 15. The sensor 8 is positioned under the pad 1 to receive pressure exerted by a patient upon movement and to be compressible relative to the applied pressure.

In use, the pump 6 delivers air to the pad 1 by means of a rotary valve 7 so that each set of cells of the pad is alternately inflated and deflated. A pressure transducer 5 is used to check the pressure of the output from the pump 6. The system operates on an inflation/deflation cycle repeating over periods varying from two minutes to over twenty minutes.

During the inflation cycle, the rotary valve 7 is in such a position that a portion of the flow goes via the tube 10 and the rest fills the cells 11 or 12 depending on the cycle. Any change in patient position or movement will cause an alteration in the airflow in the sensor pad tube 10 and will reduce or increase the differential pressure measured at the pressure transducer 16. Based on this feedback the microprocessor directly controls the power level to the pump and therefore the compressor(s) pneumatic output, thus increasing or decreasing the air flow to the cells 11, 12 to alter the amplitude of the cells 11, 12 and control the timing of the rotary valve 7 to change the timing of the inflation and deflation cycle.

The sensor air flow is measured via the differential pressure across the restrictors 15. The differential pressure is measured by pressure transducer 16 by comparison to atmospheric pressure.

The pressure recordings at the sensor exit, because of fluctuations in the air within the sensor 8, are measured and the movements analysed, the control means then controls the rotary valve 7, and thus the timing of the pressure cycle in response to the movements detected. Preferably, the detected movement values are also displayed on a display panel on the pump.

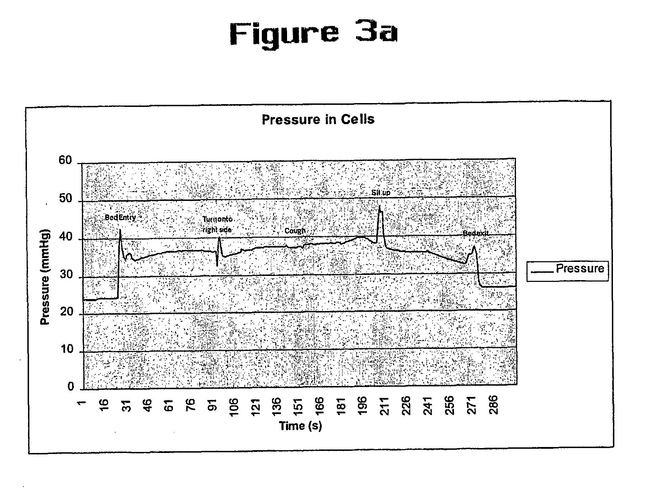

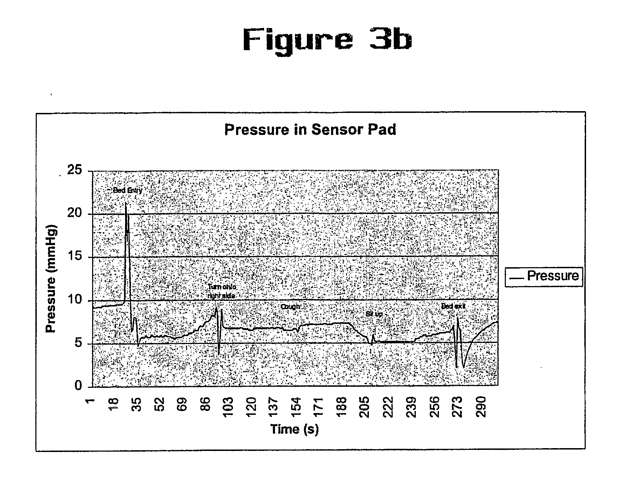

As shown in Figures 3a and 3b, the pressure transducer 16 recordings can distinguish between the various types of movements, including large and small body movements, patient exit and patient location. A windowing technique is used to detect the various movement parameters.

The large body movements indicate a significant change in body position with a subsequent redistribution of body weight. If the large body movements are within normal levels, for example, 1 large movement every 10 minutes, then the frequency of the flow cycle is reduced, increasing the comfort to the patient. The frequency is increased when there are no large body movements detected.

The patient exit is detected by sudden large changes in the pressure, or by comparison of the pressures between consecutive cycles.

Additionally, the pad 1 can be segmented into zones for a heel section, an upper leg section, a mid torso section, and a head section. The sections can be inflated at differing amplitudes for comfort and reduced risk of pressure sore development.

Although the particular embodiment described above relates to an alternating pressure pad 1, the invention applies equally to a static pressure pad with a sensor and further a pad having a zoned head, upper leg, torso and heel sections.

The pump 6 uses powered pulse width modulated (PWM) driven compressors as opposed to the mains alternating current driven compressors of the prior art. A microcontroller creates the driving waveform for the compressors C1, C2 with variable mark space constant repetition rate and constant amplitude, so that the pump 6 is not dependent for performance on any particular mains voltage or frequency. Therefore, the pump 6 can be operated from the mains voltage of any country. The compressors output is varied by varying the PWM mark space ratio from zero to maximum.

The sensor 8 and control means can be used to display the number of times the patient has moved on the support and sound an alarm if the patient has not moved or initiate contact with a third party by means of conventional communications devices.

## Claims

1. An inflatable support (1) supplied with air from a pump (6) by means of valve (7), the support (1) being an alternating inflatable support (11, 12), a sensor (8) positioned under the support (1) to measure the movements of a patient on the support (1), and control means configured to adjust the inflation and/or deflation of the support (11, 12) by the pump (6) in response to the measurement values from the sensor (8), **characterised in that** the control means is further configured to control the valve (7) to adjust the cycle times of the support (11, 12) in response to the measurement values from the sensor (8).

## Patentansprüche

1. Aufblasbare Auflage (1), der mittels eines Ventils (7) Luft aus einer Pumpe (6) zugeführt wird, wobei die Auflage (1) eine alternierend aufblasbare Auflage (11, 12) ist, einen Sensor (8), der unter der Auflage (1) angeordnet ist, um die Bewegungen eines Patienten auf der Auflage (1) zu messen, und Steuermittel, die zum Einstellen des Aufblasens und/oder Abblasens der Unterlage (11, 12) durch die Pumpe (6) als Reaktion auf die Messwerte von dem Sensor (8) konfiguriert ist, umfasst, **dadurch gekennzeichnet, dass** das Steuermittel weiter konfiguriert ist, das Ventil (7) zum Einstellen der Zykluszeiten der Auflage (11, 12) als Reaktionen auf die Messwerte von dem Sensor (8) zu steuern.

## Revendications

1. Support gonflable (1) alimenté en air depuis une pompe (6) au moyen d'une valve (7), le support (1) étant un support gonflable alternativement (11, 12), un capteur (8) situé sous le support (1) pour mesurer les mouvements d'un patient sur le support (1), et un moyen de commande configuré pour ajuster le gonflage et/ou le dégonflage du support (11, 12) par la pompe (6) en réponse aux valeurs de mesures en provenance du capteur (8), **caractérisé en ce que** le moyen de commande est, en outre, configuré pour commander la valve (7) afin d'ajuster les temps de cycles du support (11, 12) en réponse aux valeurs de mesures en provenance du capteur (8).
